# EUROPEAN PATENT SPECIFICATION

(11) **EP 4 013 484 B1**
(45) Date of publication and mention of the grant of the patent: **22.04.2026**
(21) Application number: 20853364.6
(22) Date of filing: 12.08.2020
(51) Int. Cl.: A61M 25/16

(54) **MULTI-LAYER CATHETER CONSTRUCTION**
MEHRSCHICHTIGER KATHETERAUFBAU
CONSTRUCTION DE CATHÉTER MULTICOUCHE

(30) Priority: 13.08.2019 US 201962886322 P
(43) Date of publication of application: 22.06.2022
(73) Proprietor: Shanghai Wallaby Medical Technologies Co., Inc., Pudong New Area, Shanghai 201318 (CN); Bardsley, Earl, San Clemente CA 92673 (US)
(72) Inventor: FENG, Chengcheng, Irvine, California 92620 (US); DINH, Linh, Irvine, California 92618 (US); CHOE, Jerome, Los Angeles, California 90012 (US); IAN, Cheng, Foothill Ranch, California 92610 (US); DINH, Tan, Santa Ana, California 92704 (US); SUN, Jia, Irvine, California 92602 (US); BARDSLEY, Earl, San Clemente, California 92673 (US)
(74) Representative: FRKelly
(86) International application number: PCT/US2020/045943
(87) International publication number: WO 2021/030441

(56) References cited:
- WO-A1-2017/044131
- WO-A1-2019/004100
- WO-A1-2019/115809
- CN-A- 109 498 957
- US-A1- 2003 191 451
- US-A1- 2006 095 050
- US-A1- 2009 240 235
- US-A1- 2010 268 243
- US-A1- 2013 131 641
- US-A1- 2017 020 501
- US-A1- 2018 250 498
- US-A1- 2018 250 498
- US-B2- 6 939 337

## Description

### CROSS-REFERENCE TO RELATED APPLICATION

This application is a PCT application which claims priority to and the benefit of U.S. Provisional Patent Application Serial Number 62/886,322, entitled "Multi-layer Catheter Construction," filed August 13, 2019.

### TECHNICAL FIELD

The present invention relates generally to a guide, aspiration or distal access catheter shaft design that is used for neuro vascular applications. In particular, the catheter shaft provides excellent distal flexibility, proximal stability for tracking inside the neuro vasculature, superior external lubricity, and superior interior lubricity for implant delivery.

### BACKGROUND OF THE INVENTION

Almost every neurovascular device placement or therapy takes place in or above the neck. Thus, catheters used inside a neuro vasculature must be able to transverse very tortuous anatomies. Such catheters must be designed to have a superior torquability and anti-kink characteristics within the catheter shaft, a flexible distal portion for reaching intended treatment location and to deliver the therapy without damaging the surrounding vessels. This requires a delicate balance between having the smallest possible outside profile and the largest possible internal lumen for delivery of a medical implant, movement of other catheters inside the lumen for aspiration to reperfusion occluded vessels. In addition, such a catheter must also be visibility under fluoroscopy.

In the design of catheters for neurovascular applications, the engineer must balance between functionality and construction with fundamental targets on catheter trackability, flexibility, pushability and torquability (transmittance or torsional force).

PTFE material offers excellent wear and abrasion resistance with supreme lubricity making it an ideal choice for the inner wall of the catheter. The smooth interior luminal surface of the PTFE liner reduces friction between medical device and catheter as the device is pushed through the tight confines of the catheter lumen. Dip coating and extrusion are two common manufacturing method for making PTFE liner. The Dip coating process is one method used for attaining a PTFE liner. This process can be performed onto a mandrel as part of the catheter shaft construction process. Once the coating has cured, the additional components of the catheter, for example, the nylon jacketing, braiding are built upon the cured dip-coated mandrel. Dip-coating, while appearing to be a simple process at the outset, has some limitations. Dip coating can experience unevenness resembling the surface of an orange peel. Sometimes, dip-coated surfaces can have the appearance of many cross-sectional lines called chatter resulting from vibrations during the coating process. Dip-coated surfaces can also have craters, depressions, or even holes in the cured layers caused by contaminants - including moisture - during the coating process. All these defects will have a negative impact on interior lubricity of the catheter lumen and therefore gravely impede catheter's use. While these defects can be addressed in different ways, the superior precision of the dip-coating process will typically entail greater cost and time.

Extruded PTFE tubing is another method of manufacturing PTFE catheter inner liner. Although extruded PTFE has the lowest coefficient of friction, a typical extruded PTFE tubing has a wall thickness of around 0.001". This wall thickness could lead to an overall increase in stiffness of the catheter, which is not ideal for traveling through the tortuous path of the neuro vasculature.

US patent application number US 2017/0020501 A1 discloses an introducer sheath with a braided filament securement mechanism.

An ideal catheter for neurovascular application should have a minimum outer diameter for traveling through small vasculature, and a maximum internal diameter for delivery medical device/implant. This inevitably leads to a need for an ultra-thin catheter inner liner. However, with an ultra-thin catheter inner liner, the engineer will need to incorporate a shaft support component, such as metal wire layer covering the inner catheter liner either in braid profile or in coil profile, to enhance pushability and the kink resistance of the catheter. Minimum contact pressure is required between the metal wire layer and the inner catheter liner in order to maintain the integrity of the catheter construction. Often times, such minimum contract pressure leads to an increase in interior surface roughness of the inner catheter liner (20), and significantly reduce the interior surface lubricity.

Thus there is a need to develop a catheter for neurovascular application which will satisfy the goal of elevated pushability, trackability, flexibility, and torquability, but also a large lumen is needed to achieve maximized aspiration flow; a smaller outside diameter to allow the catheter to track into smaller vessels without posing additional vessel damage to the target treatment location; outer surface lubricity; and a supreme interior surface lubricity to reduce the deployment force. These objectives are achieved by means of a catheter shaft according to the invention as defined in claim 1. Preferred embodiments are defined in the dependent claims.

### SUMMARY

An aspect of the present teachings provides a catheter shaft for delivering and deploying a medical device. According to the invention the catheter shaft comprises an ultra-thin inner catheter liner having a luminal wall thickness and being made of expanded PTFE tubing, a first coil middle layer, a second braid middle layer, and an outer jacket layer. The first coil middle layer is made of a flat wire with a width to thickness ratio of at least 2:1. The outer jacket layer is made of materials comprising a soft durometer material forming a distal portion of the outer jacket, a medium durometer material forming a middle portion of the outer jacket, and a strong durometer material forming a proximal portion of the outer jacket.

One embodiment of the present teachings provides that the inner catheter liner of the catheter shaft has an outer diameter to thickness ratio of at least 10:1. The inner catheter liner has a minimum inner diameter of 0.8 mm. One embodiment of the present teachings provides that the first coil middle layer of the catheter shaft has a maximum thickness of 0.06 mm. Another embodiment of the present teachings provides that the first coil middle layer of the catheter shaft has a minimum width of 0.08 mm.

One embodiment of the present teachings provides that the first coil middle layer of the catheter shaft covers at least 30% area of an exterior luminal surface of the inner catheter liner. In another embodiment, depending on the desired stiffness, the coverage area between the first coil middle layer of the catheter shaft over the exterior luminal surface of the inner catheter line ranges between 30-75%. One skilled in the art should understand that the less the coverage area between the first coil middle layer and the inner catheter liner, the more flexible the overall catheter.

One embodiment of the present teachings provides that the second braid middle layer (40) of the catheter shaft has a maximum thickness of 0.03 mm.

One embodiment of the present teachings provides that the inner catheter liner and the outer jacket layer of the catheter shaft are of the same length. Another embodiment of the present teachings provides the first coil middle layer and the second braid middle layer are encapsulated inside both the inner catheter liner and the outer jacket layer.

One embodiment of the present teachings provides that the outer jacket layer bonds with the inner catheter liner through a gap between the wires forming the first coil middle layer (30) and the second braid middle layer.

One embodiment of the present teachings provides that the first coil middle layer of the catheter shaft is made of at least two wires wound in a same direction. Another embodiment of the present teachings provides that the first coil middle layer of the catheter shaft is made of at least two wires wound in an opposite direction.

One embodiment of the present teachings provides that the second braid middle layer (40) placed in between the first coil middle layer and the outer jacket layer. Another embodiment of the present teachings provides that the second braid middle layer is placed over at least a proximal and a middle portion of the first coil middle layer. Another embodiment of the present teachings provides that the second braid middle layer is at least 20 mm shorter than the first coil middle layer.

One embodiment of the present teachings provides that the catheter shaft has an outer diameter to an inner luminal dimeter ratio of at least 1.2:1.

### BRIEF DESCRIPTION OF THE DRAWINGS

Fig. 1A a perspective view of a catheter having a catheter shaft and a proximal control mechanism in accordance with the present teachings;
Fig. 1B is a perspective view of an exemplary embodiment of the multi-layer catheter shaft construction in accordance with the present teachings;
Fig. 2A is a perspective view of an exemplary first coil middle layer of the catheter shaft in accordance with the present teachings;
Fig. 2B is a perspective view of an exemplary first coil middle layer of the catheter shaft in accordance with the present teachings;
Fig. 2C is a perspective view of an exemplary first coil middle layer of the catheter shaft in accordance with the present teachings;
Fig. 3 is a perspective view of an exemplary second braid middle layer of the catheter shaft in accordance with the present teachings;
Fig. 4 is a perspective view of an exemplary outer jacket layer of the catheter shaft in accordance with the present teachings.

### DETAILED DESCRIPTION

In the description certain references are made to non-SI units (inches). These are to be translated to corresponding SI units (mm) using the factor: 1 inch = approximately 25.4 mm.

Certain specific details are set forth in the following description and figures to provide an understanding of various embodiments of the present teachings. Those of ordinary skill in the relevant art would understand that they can practice other embodiments of the present teachings without one or more of the details described herein. Thus, it is not the intention of the applicant(s) to restrict or in any way limit the scope of the appended claims to such details. While various processes are described with reference to steps and sequences in the following disclosure, the steps, and sequences of steps should not be taken as required to practice all embodiments of the present teachings.

As used herein, the term "lumen" means a canal, a duct, or a generally tubular space or cavity in the body of a subject, including veins, arteries, blood vessels, capillaries, intestines, and the like. The term "lumen" can also refer to a tubular space in a catheter, a sheath, a hollow needle, a tube, or the like.

As used herein, the term "proximal" shall mean close to the operator (less into the body) and "distal" shall mean away from the operator (further into the body). In positioning a medical device inside a patient, "distal" refers to the direction relatively away from a catheter insertion location and "proximal" refers to the direction relatively close to the insertion location.

As used herein, the term "wire" can be a strand, a cord, a fiber, a yarn, a filament, a cable, a thread, or the like, and these terms may be used interchangeably.

As used herein, the term "sheath" may also be described as a "catheter" and, thus, these terms can be used interchangeably.

Unless otherwise specified, all numbers expressing quantities, measurements, and other properties or parameters used in the specification and claims are to be understood as being modified in all instances by the term "about." Accordingly, unless otherwise indicated, it should be understood that the numerical parameters set forth in the following specification and appended claims are approximations. At the very least and not as an attempt to limit the application of the doctrine of equivalents to the scope of the claims, numerical parameters should be read in light of the number of reported significant digits and the application of ordinary rounding techniques.

It will be understood that the words "comprising" (and any form of comprising, such as "comprise" and "comprises"), "having" (and any form of having, such as "have" and "has"), "including" (and any form of including, such as "includes" and "include") or "containing" (and any form of containing, such as "contains" and "contain") when used herein, specify the presence of stated features, integers, steps, operations, elements, and/or components, but do not preclude the presence or addition of one or more other features, integers, steps, operations, elements, components, and/or groups thereof.

It will be further understood that, although the terms first, second, third, etc. may be used herein to describe various limitations, elements, components, regions, layers and/or sections, these limitations, elements, components, regions, layers and/or sections should not be limited by these terms. These terms are only used to distinguish one limitation, element, component, region, layer or section from another limitation, element, component, region, layer or section. Thus, a first limitation, element, component, region, layer or section discussed below could be termed a second limitation, element, component, region, layer or section without departing from the teachings of the present application.

It will be further understood that when an element is referred to as being "on", "attached", "connected" or "coupled" to another element, it can be directly on or above, or connected or coupled to, the other element or one or more intervening elements can be present. In contrast, when an element is referred to as being "directly on", "directly attached", "directly connected" or "directly coupled" to another element, there are no intervening elements present. Other words used to describe the relationship between elements should be interpreted in a like fashion (e.g. "between" versus "directly between," "adjacent" versus "directly adjacent," etc.).

It will be further understood that when a first element is referred to as being "in", "on" and/or "within" a second element, the first element can be positioned: within an internal space of the second element, within a portion of the second element (e.g. within a wall of the second element); positioned on an external and/or internal surface of the second element; and combinations of one or more of these.

Spatially relative terms, such as "beneath," "below," "lower," "above," "upper" and the like may be used to describe an element and/or feature's relationship to another element(s) and/or feature(s) as, for example, illustrated in the figures. It will be further understood that the spatially relative terms are intended to encompass different orientations of the device in use and/or operation in addition to the orientation depicted in the figures. For example, if the device in a figure is turned over, elements described as "below" and/or "beneath" other elements or features would then be oriented "above" the other elements or features. The device can be otherwise oriented (e.g. rotated 90 degrees or at other orientations) and the spatially relative descriptors used herein interpreted accordingly.

The terms "reduce", "reducing", "reduction" and the like, where used herein, are to include a reduction in a quantity, including a reduction to zero. Reducing the likelihood of an occurrence shall include prevention of the occurrence.

The term "and/or" where used herein is to be taken as specific disclosure of each of the two specified features or components with or without the other. For example "A and/or B" is to be taken as specific disclosure of each of (i) A, (ii) B and (iii) A and B, just as if each is set out individually herein.

The term "diameter" where used herein to describe a non-circular geometry is to be taken as the diameter of a hypothetical circle approximating the geometry being described. For example, when describing a cross-section, such as the cross-section of a component, the term "diameter" shall be taken to represent the diameter of a hypothetical circle with the same cross-sectional area as the cross-section of the component being described.

The terms "major axis" and "minor axis" of a component where used herein are the length and diameter, respectively, of the smallest volume hypothetical cylinder which can completely surround the component.

It is appreciated that certain features of the invention, which are, for clarity, described in the context of separate embodiments, may also be provided in combination in a single embodiment. Conversely, various features of the invention which are, for brevity, described in the context of a single embodiment, may also be provided separately or in any suitable subcombination. For example, it will be appreciated that all features set out in any of the claims (whether independent or dependent) can be combined in any given way.

The present teaching relates to a catheter shaft having a single lumen, large-bore, and a thin catheter wall. According to one embodiment of the present teaching, the disclosed catheter configuration offers excellent distal flexibility, an outstanding proximal pushability and torquability, superior external lubricity, and superior internal surface lubricity.

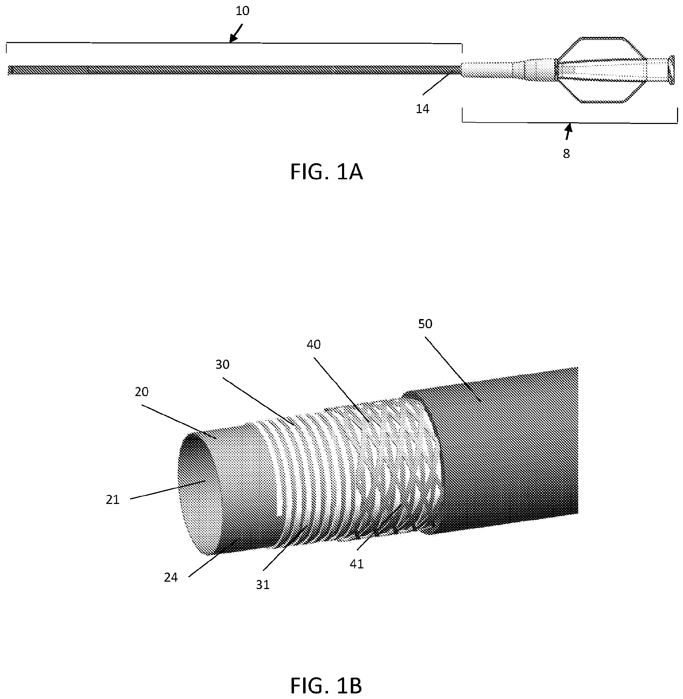

As illustrated in Figs. 1A-1B, the catheter shaft (10) has a four-layer construction. An inner layer (20) of the catheter shaft (10) includes a full-length ultra-thin inner catheter liner (20) with a central lumen (21) extending from one end to another. A first middle layer (30) of the catheter shaft (10) includes a flat wire coil (31) winding over the exterior luminal surface (24) of the inner catheter liner (20). A second middle layer (40) of the catheter shaft (10) includes a wire braid placing over the exterior luminal surface of the first coil middle layer (30). An outer layer of the catheter shaft (10) includes a tubular jacket (50) placing over the second braid middle layer (40) of the catheter shaft (10) and being melted and bound with the inner layer (20) of the catheter shaft (10) through the two middle layers (30, 40) of the catheter. In addition, according to one embodiment of the present teaching, outer (50) and inner (20) layers of the catheter shaft (10) have a generally same length, with the two middle layers (30, 40) of the catheter shaft (10) slightly shorter than both the outer (50) and inner layers (20) so as to be encapsulated between and within the outer (50) and inner (20) layers of the catheter shaft (10).

Continue referring to Fig. 1A, a radiopaque marker band is fixed at the distal tip of the catheter shaft (10) assembly to allow visibility through fluoroscopy. Fig. 1A also illustrates that the proximal end (14) of the catheter shaft (10) connects to the catheter proximal control mechanism (8). Specific proximal control mechanism design varies according to the intended application. Thus, Fig. 1A is merely an example for the purpose of explaining the present teaching.

According to one embodiment of the present teaching, the catheter shaft (10) assembly has a general length of 1100-1400 mm, an outer diameter of 1.4-2.7 mm, an inner lumen diameter of 1.2-2.3 mm. One skilled in the art should understand that overall length and size of the catheter shaft (10) assembly could be easily modified to fit for a specific application. Thus, the number disclosed herein should be considered as a reference, and should not be viewed as limiting to the scope of the claim.

Referring to Fig. 1B, according to one embodiment of the present teaching, the inner layer (20) of the catheter shaft (10) has a tubular profile with a central lumen (21) extending from one end to another. According to one embodiment of the present teaching, the inner layer (20) of the catheter shaft (10) has a length of 1150-1400 mm, an outer diameter of 1.75-1.85 mm, an inner luminal diameter of 1.73-1.83 mm, a luminal wall thickness of 0.013 mm - 0.025 mm (or 0.0005" to 0.001"). According to one embodiment of the present teaching, the inner layer (20) of the catheter shaft (10) is designed to have a relatively thin luminal wall. Doing so, not only would reduce the overall profile of the catheter shaft (10), but also improve the overall flexibility of the catheter shaft (10).

According to one embodiment, the internal luminal surface of the inner layer (20) of the catheter shaft (10) directly contacts a medical device/implant as it travels through the catheter. Thus the internal luminal surface of this inner layer (20) of the catheter shaft (10) is designed to provide sufficient lubricity which minimizes a force required to push the device/implant through the catheter lumen (21).

According to one embodiment not falling under the invention as claimed, the inner layer (20) of the catheter shaft (10) is made of extruded PTFE (polytetrafluoroethylene). According to the invention, the inner layer (20) of the catheter shaft (10) is made of expanded PTFE, also known as ePTFE. ePTFE inner catheter liner (20) is made by expanding PTFE tubing carefully using a non-standard manufacturing process such as heating an extruded PTFE tube to the desired temperature followed by enlarge its inner diameter over a core mandrel. Such a post-extrusion process will produce an enlarged inner tube diameter and an ultrathin tubular wall with microscopic pores. The resulted ePTFE inner catheter liner (20) has the physical character of air-permeable, soft & flexible, and feels somewhat like smooth, spongey, like a membrane. The resulted ePTFE inner catheter liner (20) also has the character of high linear strength, chemically inert, remains watertight at low pressure, has a low dielectric constant, and offers excellent radial expansion and UV resistance.

One skilled in the art should understand that although extruded PTFE , not according to the invention as claimed, and expanded PTFE (ePTFE) tubing , according to the invention, have been described here for the purpose of explaining present teaching, according to further embodiments not falling under the invention as claimed the inner layer (20) of the catheter shaft (10) could be made with other material, such as FEP (fluorinated ethylene propylene), FEP (fluorinated ethylene propylene), HDPE (high-density polyethylene), LDPE (low-density polyethylene), Polyethylene terephthalate (PET), Polypropylene (PP), Polyamides/Pebax, with any manufacturing process known in the field such as direct extrusion and/or post extrusion axial stretching and/or radial expansion in order to achieve an ultra-thin luminal wall.

In an alternative embodiment of the present teaching not falling under the invention as claimed, the inner layer could be made of two materials joined together. For example, a distal portion of the inner layer could be made of polyurethane tubing while the rest portion of the inner layer is made of extruded PTFE. The extruded PTFE provides the catheter shaft with pushability, and the polyurethane distal portion provides the catheter shaft with flexibility and soft distal tip to better tracking insider the neurovasculature. According to one example embodiment, the distal polyurethane portion of the inner layer could be made of polyurethane tube with a luminal wall thickness of 0.013 mm - 0.025 mm (or 0.0005" to 0.001"). Such distal polyurethane tubing then joins to the rest of the PTFE portion by thermal sealing, or other known method in the field. Although polyurethane and PTFE combination are disclosed here as one exemplary inner layer construction, one skilled in the art should understand that other material combination could be incorporated to achieve the design purpose of the present teaching which is an inner layer of the catheter shaft with ultra-thin luminal wall and a smooth and lubricate inner luminal surface in order to provide a good tractability for minim invasive procedure.

Continue referring to Fig. 1B, the first middle layer (30) of the catheter shaft (10) includes a wire coil (31) winding over the exterior luminal surface of the inner catheter liner (20). The wire coil middle layer (30) is added to as a support to inner catheter liner (20), to enhance distal lateral flexibility while adding hoop strength for even better kink-resistance. According to one embodiment, the wire used for forming the coil middle layer (30) of the catheter shaft (10) assembly has a generally flat configuration. Using a flat wire over the inner layer provides a smooth inner luminal surface since the contact pressure between the flat wire and the inner layer is low. As a result of low contact pressure, deformation and impression formed by the coil layer onto the inner layer is reduced. With the incorporation of the flat wire coil, the inner layer of the catheter shaft could adapt an ultra-thin luminal wall thickness. A catheter shaft with flat wire and ultra-thin inner layer construction provides a desired inner diameter for implant delivery while maintaining a smaller outer profile for better performance inside the vasculature.

One skilled in the art should understand Fig. 1B is provided for the purpose of illustrating and explaining the four-layer construction of the catheter shaft according to one embodiment of the present teaching. Fig. 1B does not intend to be an actual presentation of the length or relative length of each layers of the catheter shaft.

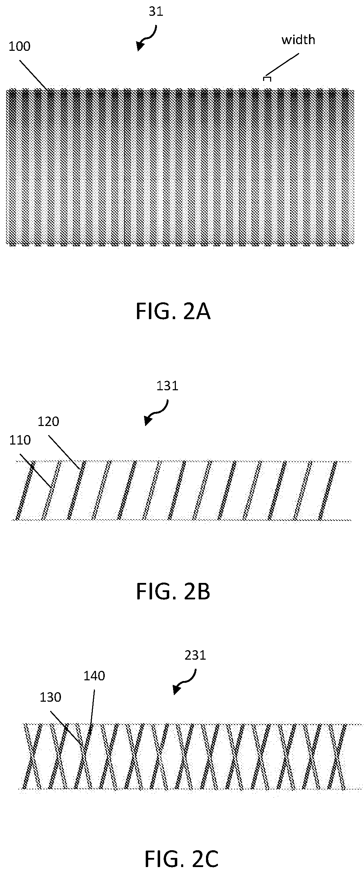

That is, as shown in Fig. 2A, the cross-section of the wire (100) has a greater width than its thickness. Thus, this embodiment creates a coil middle layer (30) with a maximum contact area with the inner layer (20) of the catheter shaft (10). Since the inner layer (20) of the catheter shaft (10) has an ultra-thin luminal wall, the greater contact area between the coil middle layer (30) and the outer luminal surface of the inner catheter layer (20), the smoother the inner layer (20) luminal surface. One skilled in the art should understand that with the same coil width, the greater the coil thickness, the better the kink resistance. Thus a design trade-off must be achieve between the adequate kink resistance and a small overall profile of the catheter shaft (10).

According to one embodiment, and as shown in Fig. 2A, the wire coil (31) has a 0.04-0.11 mm width and a 0.02-0.06 mm thickness. In one embodiment, the wire coil (31) has a width of 0.003 inch and a thickness of 0.0015 inch. According to another embodiment of the present teaching, the cross-sectional area of the wire (100) forming the coil middle layer (30) is 0.001-0.007 mm² with the width to thickness ratio of at least 2:1. According to one embodiment, the first coil middle layer (30) covers at least 38% area of the exterior luminal surface of the inner catheter layer (20). According to yet another embodiment of the present teaching, the coil (31) has a pitch of 0.12-0.25mm with a minimum gap of 0.025 mm in between each helical turn. In another embodiment, the coil (31) could be wound in a left hand or a right-hand direction.

According to another embodiment of the present teaching, the first middle layer (30) is a helical coil (31) with a constant pitch. According to another embodiment of the present teaching, the wire (100) forming the coil middle layer (30) has a constant thickness and a constant width throughout its entire length. Yet in another embodiment of the present teaching, in order to achieve a desired steerability of the catheter shaft (10) while achieving a greater flexibility at the distal section of the catheter shaft (10), the helical coil (31) could have a smaller pitch at a proximal portion of the catheter shaft (10), and a greater pitch at a distal portion of the catheter, and a gradual change in between. In Another embodiment of the present teaching, while the thickness of the wire remaining the same, the wire (100) forming the helical coil (31) has a greater width at a proximal portion of the catheter shaft (10), and a smaller width profile at a distal portion of the catheter shaft (10), and a gradual change in between. Thus, a specific embodiment explained and illustrated herein used to limit the scope of the present teaching.

One skilled in the art should understand that comparing to round wire coil, the flat wire coil, when the flat wire having the same cross sectional area as the round wire, offers at least the same kink resistance as the round wire coil, but with at least a 33% reduction in the overall wall thickness profile. Thus the embodiment in present teaching provide a minimum increase in catheter radial profile. In another word, the present teaching offers a largest possible inner catheter lumen size while keeping the outer radial catheter profile as minimum as possible.

According to one embodiment, the flat wire that forms the coil middle layer (30) could be formed with Stainless Steel, Tungsten, Cobalt Chromium Alloys, and others. In another embodiment, the flat wire (100) that forms the coil middle layer (30) could be formed with super elastic material such Nitinol. In one embodiment, Nitinol could allow the coil middle layer (30) to recover its pre-configured circular lumen shape even when catheter is kinked during use.

Fig. 2A illustrates a single wire helical coil (31). One skilled in the art should know that the multi-wire coil (131, 231) could also be incorporated into the construction of the first coil middle layer (30). For example, as illustrated in Fig. 2B, the multi-wire coil (131) forming the coil middle layer (30) could be made of two wires (110, 120), each of a different material, wind in the same direction. Doing so, the coil middle layer (30) could achieve a combined strength and other mechanical properties of the two materials. Another exemplary embodiment as shown in Fig. 2C, the multi-wire coil (231) forming the coil middle layer (30) could be made of two wires (130, 140), each of a different material, wind in the opposite direction. According to one embodiment, for both the exemplary embodiments shown in Figs 2B-2C, the two wires (130, 140) could wind alternatively so that they are substantially in the same layer. In another embodiment, the one wire could wind on top of the coil formed by the other wire. One skilled in the art should understand, each wire (110, 120, 130, 140) configuration including its width and thickness, as well as the coil pitch and winding angle, can all be independently adjusted to achieve the optimum result. According to one embodiment, the multi-wire coil (131, 231) comprises 2-4 wires. In another embodiment, the multi-wire coil (131, 231) could be wound in a left hand or a right-hand direction. In another embodiment, the multi- wire coil (131, 231) could have a pitch of 0.07-0.5mm. In yet other embodiments, the wire used in the multi-wire coil tubing construction ranges from 0.04-0.11 mm in width and 0.02-0.06 mm in thickness.

Continue referring to Fig. 1B, according to one embodiment, a second middle layer (40) is placed over the exterior of the first coil middle layer (30). The second middle layer (40) is made of a tubular wire braid (41). Said tubular wire braid (41) enhances the axial and torsional control of the catheter shaft (10); reduces or virtually eliminates lag associated with push/pull or rotational input by the clinician.

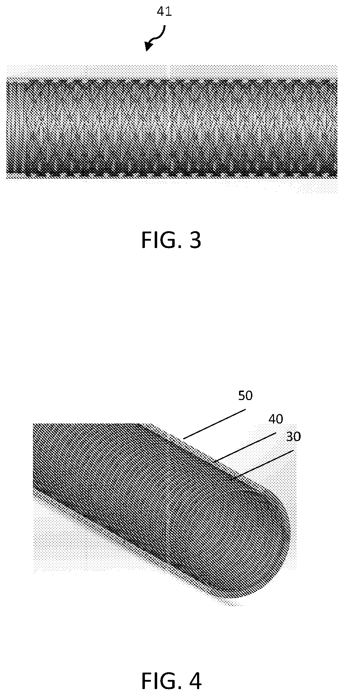

Fig. 3 illustrates one exemplary braiding pattern. According to one embodiment of the present teaching, the tubular wire braid (41) of the second braid middle layer (40) is made with 16 wires and a regular braid pattern. In another embodiment, the second braid middle layer (40) could be made with 16 wires and a regular diamond braid pattern which tends to provide better torque and more kink resistance. One skilled in the art should understand that braid pattern and numbers of wire used for braid could be changes. Thus, what disclosed here should not be used to limit the scope of the present teaching.

According to one embodiment of the present teaching, the second middle layer (40) is placed over the exterior of the first coil middle layer (30), as illustrated in Fig. 1B. According to one embodiment of the present teaching, the second braid middle layer (40) has a general thickness of 0.025-0.066 mm with each wire making the braid having an outer thickness of 0.013-0.0033 mm. According to one embodiment of the present teaching, the second braid middle layer (40) has a length of 1150-1400 mm, substantially similar to the length of the first coil middle layer (30). According to another embodiment, the second braid middle layer (40) has a length of 1100-1350 mm, significantly shorter than the first coil middle layer (30) as such the second braid middle layer (40) only cover middle and proximal portions of the first coil middle layer (30) without covering the distal portion of the first coil middle layer (30). Yet according to another embodiment, the second braid middle layer (40) is shorter than the first coil middle layer (30) by 2cm at the distal end of the first coil middle layer (30) as shown in Fig. 1B.

Continue referring to Fig. 1B, according to one embodiment, a polymer jacket (50) places the second braid middle layer (40) forming an outer layer of the catheter. According to one embodiment, the outer jacket (50) has an original tubular configuration, which laid down over the second braid middle layer (40). The entire catheter shaft (10) assembly is then subject to a reflow process, whereby the outer jacket (50) and/or the inner liner materials are melted to build a composite catheter shaft (10). According to one embodiment of the present teaching, the outer jacket layer (50) material melts and flow through the gap between wires of the second braid (40) and first coil middle layer (30), and adhere to the inner layer (20) of the catheter shaft (10) underneath. One skilled in the art should understand that the temperature used for melting the outer jacket layer (50) in order to form a bonding with the inner layer (20) of the catheter shaft (10) is balanced to allow sufficient material flow through the gap between wire, without introducing grooves and valley to the interior luminal surface of the inner layer (20) of the catheter shaft (10) which would affect the smoothness and lubricity of the catheter lumen.

According to one embodiment, the outer jacket layer (50) is made of a single material, such as nylon or PEBAX, having a constant durometer. According to another embodiment of present teaching, the outer jacket layer (50) has a durometer of 25-80 shore D, a length of 20-500 mm, an outer diameter of 2.0-2.5 mm, and a wall thickness of 0.025-0.13 mm. According to one embodiment, the outer jacket layer (50) covers the entire length of the two middle layers (30, 40) and the entire length of the inner layer (20) of the catheter shaft (10).

Now referring to Fig. 4, where another embodiment of the outer jacket layer (50) is presented. According to one embodiment, the outer jacket layer (50) has multiple sections each made of a different material having a different durometer. For example, as illustrated in Fig. 4, a distal portion of the outer jacket layer (50) is made of super-low durometer material such as TPU with a durometer about Shore 60A. A proximal portion of the outer jacket layer (50) is made of relatively high durometer material such as Nylon with a durometer about Shore 75D. A transitional portion in between the distal and proximal portions of the outer jacket layer (50) is made of relatively high durometer material such as PEBAX with durometer ranging from Shore 30D-63D. Accordingly, the low durometer distal outer layer provides the catheter shaft (10) assembly with superior flexibility for traveling through a tortuous path of the small vasculature. The high durometer proximal portion of the outer jacket layer (50) provides the catheter shaft (10) assembly with stiffness and superior controllability. And the transitional portion allows a gradually shifts from the distal ultrasoft portion to a proximal stiff and supportive portion.

Continue referring to Fig. 4, according to one embodiment, the distal portion of the outer jacket layer (50) has a length of 100-200 mm, the transitional portion of the outer jacket layer (50) has a length of 10-30 mm, and the proximal portion of the outer jacket layer (50) has a length of 1000-1200 mm. According to one embodiment of the present teaching, both distal, transitional, and proximal portion of the outer jacket layer (50) has substantially similar thickens.

According to another embodiment of the present teaching, additional coating layer can be added to the catheter construct described in the present teaching. For example, a hydrophilic coating in order to achieve a smooth and slippery surface, which allows the catheter to track in the vasculature with ease.

Although not specifically described in the above disclosure, one skilled in the art should understand that one or more radiopaque markers are used to aid visualization through imaging devices such as fluoroscope; X-ray; CT scanner; MRI; ultrasound imager. A marker, as disclosed herein, can be applied to any part of the catheter shaft (10) of the present teachings. A radiopaque marker can be, adhered, swaged, crimped, otherwise placed, and secured in or on the catheter shaft (10). The radiopaque marker may be made of tantalum, tungsten, platinum, iridium, gold, or alloys of these materials or other materials that are known to those skilled in the art. The radiopaque marker can also be made of numerous paramagnetic materials, including one or more elements with atomic numbers 21-29, 42, 44, and 58-70, such as chromium (III), manganese (II), iron (III), iron (II), cobalt (II), copper (II), nickel (II), praseodymium (III), neodymium (III), samarium (III), ytterbium (III), gadolinium (III), terbium (III), dysprosium (III), holmium (III) and erbium (III), or other MR visible materials that are known to those skilled in the arts.

One skilled in the art should understand, although most exemplary embodiments described above refer to a catheter shaft (10) used for neuro vasculature application, exemplary embodiments of the present teaching could be used in any other suitable minimum invasive application. For example, the exemplary embodiments of the present teaching could be used to deliver vaso-occlusive devices, stents, aspiration, stent retrievers for ischemic stroke, and etc. In another example, an exemplary embodiment of the present teaching could be used to precisely deliver an implant into an aneurysm, such as a brain aneurysm. In another example, the exemplary embodiment of the present teaching could be used to precisely delivery an implant into a blood vessel such as a blood vessel of the brain, a patent blood vessel, or other locations.

The foregoing description and accompanying drawings set forth a number of examples of representative embodiments at the present time. Various modifications and alternative designs will become apparent to those skilled in the art in light of the foregoing teachings .

## Claims

1. A catheter shaft (10) for delivering and deploying a medical device, the catheter shaft comprising
an ultra-thin inner catheter layer (20), a first coil middle layer (30), Z a second braid middle layer (40), and an outer jacket layer (50),
wherein the ultra-thin inner catheter layer has a luminal wall thickness and is made of expanded PTFE tubing,
wherein the first coil middle layer is made of a flat wire (31) with a width to thickness ratio of al least 2:1,
wherein the outer jacket layer is made of a plurality of materials with a soft durometer material forming a distal portion of the outer jacket, a range of medium durometer materials forming a middle portion of the outer jacket, and a hard durometer material forming a proximal portion of the outer jacket.

2. The catheter shaft of claim 1, wherein the first coil middle layer has a maximum thickness of 0.06 mm.

3. The catheter shaft of claim 1, wherein the second braid middle layer has a diamond braid pattern.

4. The catheter shaft of claim 1, wherein the first coil middle layer covers at least 30% area of an exterior luminal surface of the inner catheter layer.

5. The catheter shaft of claim 1, wherein the second braid middle layer has a maximum thickness of 0.066 mm.

6. The catheter shaft of claim 1, wherein the inner catheter layer and the outer jacket layer are of the same length.

7. The catheter shaft of claim 1, wherein the first coil middle layer and second braid middle layer are encapsulated inside both the inner catheter layer and the outer jacket layer.

8. The catheter shaft of claim 1, wherein the first coil middle layer is made of at least two wires wound in a same or an opposite direction.

9. The catheter shaft of claim 1, wherein the second braid middle layer placed in between the first coil middle layer and the outer jacket layer.

10. The catheter shaft of claim Z 9, wherein the second braid middle layer is placed over at least a proximal and a middle portion of the first coil middle layer, optionally wherein the second braid middle layer is at least 20 mm shorter than the middle portion of the first coil middle layer.

11. The catheter shaft of claim 1, wherein the catheter shaft has an outer diameter to an inner luminal dimeter ratio of at least 1.2:1.

12. The catheter shaft of claim 1, wherein the first coil middle layer has a smaller pitch at a proximal portion of the catheter shaft, and a greater pitch at a distal portion of the catheter, and a gradual change in between.

13. The catheter shaft of claim 1, wherein the first coil middle layer is placed over the ultra-thin inner catheter layer, the second braid middle layer is placed over the first coil middle layer, and the outer jacket layer is placed over the second braid middle layer and wherein the outer jacket layer is melted and bound with the ultra-thin inner layer through the first coil middle layer and second braid middle layer.

## Patentansprüche

1. Katheterschaft (10) zum Zuführen und Einsetzen einer medizinischen Vorrichtung, wobei der Katheterschaft Folgendes umfasst:
eine ultradünne innere Katheterschicht (20), eine erste Spulenmittelschicht (30), eine zweite Geflechtmittelschicht (40) und eine äußere Mantelschicht (50),
wobei die ultradünne innere Katheterschicht eine luminale Wandstärke aufweist und aus einem expandierten Polytetrafluorethylen-Schlauch (PTFE-Schlauch) hergestellt ist,
wobei die erste Spulenmittelschicht aus einem Flachdraht (31) mit einem Verhältnis von Breite zu Dicke von mindestens 2:1 hergestellt ist,
wobei die äußere Mantelschicht aus einer Vielzahl von Materialien hergestellt ist, wobei ein weiches Durometermaterial einen distalen Abschnitt des äußeren Mantels bildet, eine Reihe von Materialien mit mittlerer Durometerhärte einen mittleren Abschnitt des äußeren Mantels bildet und ein hartes Durometermaterial einen proximalen Abschnitt des äußeren Mantels bildet.

2. Katheterschaft nach Anspruch 1, wobei die erste Spulenmittelschicht eine maximale Dicke von 0,06 mm aufweist.

3. Katheterschaft nach Anspruch 1, wobei die zweite Geflechtmittelschicht ein Rautengeflechtmuster aufweist.

4. Katheterschaft nach Anspruch 1, wobei die erste Spulenmittelschicht mindestens 30 % der Fläche einer äußeren luminalen Oberfläche der inneren Katheterschicht bedeckt.

5. Katheterschaft nach Anspruch 1, wobei die zweite Geflechtmittelschicht eine maximale Dicke von 0,066 mm aufweist.

6. Katheterschaft nach Anspruch 1, wobei die innere Katheterschicht und die äußere Mantelschicht die gleiche Länge aufweisen.

7. Katheterschaft nach Anspruch 1, wobei die erste Spulenmittelschicht und die zweite Geflechtmittelschicht sowohl innerhalb der inneren Katheterschicht als auch der äußeren Mantelschicht eingekapselt sind.

8. Katheterschaft nach Anspruch 1, wobei die erste Spulenmittelschicht aus mindestens zwei Drähten hergestellt ist, die in einer gleichen oder einer entgegengesetzten Richtung gewickelt sind.

9. Katheterschaft nach Anspruch 1, wobei die zweite Geflechtmittelschicht zwischen der ersten Spulenmittelschicht und der äußeren Mantelschicht platziert ist.

10. Katheterschaft nach Anspruch 9, wobei die zweite Geflechtmittelschicht über mindestens einem proximalen und einem mittleren Abschnitt der ersten Spulenmittelschicht platziert ist, gegebenenfalls wobei die zweite Geflechtmittelschicht mindestens 20 mm kürzer als der mittlere Abschnitt der ersten Spulenmittelschicht ist.

11. Katheterschaft nach Anspruch 1, wobei der Katheterschaft ein Verhältnis von Außendurchmesser zu innerem luminalen Durchmesser von mindestens 1,2:1 aufweist.

12. Katheterschaft nach Anspruch 1, wobei die erste Spulenmittelschicht eine kleinere Steigung an einem proximalen Abschnitt des Katheterschafts und eine größere Steigung an einem distalen Abschnitt des Katheters und eine allmähliche Änderung dazwischen aufweist.

13. Katheterschaft nach Anspruch 1, wobei die erste Spulenmittelschicht über der ultradünnen inneren Katheterschicht platziert ist, die zweite Geflechtmittelschicht über der ersten Spulenmittelschicht platziert ist und die äußere Mantelschicht über der zweiten Geflechtmittelschicht platziert ist und wobei die äußere Mantelschicht geschmolzen und durch die erste Spulenmittelschicht und die zweite Geflechtmittelschicht hindurch mit der ultradünnen inneren Schicht verbunden ist.

## Revendications

1. Tige de cathéter (10) destinée à l'acheminement et au déploiement d'un dispositif médical, la tige de cathéter comprenant :
une couche de cathéter interne ultra-mince (20), une première couche intermédiaire en spire (30), une seconde couche intermédiaire tressée (40) et une couche de gaine externe (50),
dans laquelle la couche de cathéter interne ultra-mince présente une épaisseur de paroi luminale et est constituée d'un tube en PTFE expansé,
dans laquelle la première couche intermédiaire en spire est constituée d'un fil plat (31) dont un rapport largeur/épaisseur est d'au moins 2:1,
dans laquelle la couche de gaine externe est constituée d'une pluralité de matériaux, un matériau de faible dureté formant une partie distale de la gaine externe, une gamme de matériaux de dureté intermédiaire formant une partie médiane de la gaine externe et un matériau de dureté élevée formant une partie proximale de la gaine externe.

2. Tige de cathéter selon la revendication 1, dans laquelle la première couche intermédiaire en spire présente une épaisseur maximale de 0,06 mm.

3. Tige de cathéter selon la revendication 1, dans laquelle la seconde couche intermédiaire tressée présente un motif de tressage en losange.

4. Tige de cathéter selon la revendication 1, dans laquelle la première couche intermédiaire en spire couvre au moins 30 % de la surface luminale externe de la couche de cathéter interne.

5. Tige de cathéter selon la revendication 1, dans laquelle la seconde couche intermédiaire tressée présente une épaisseur maximale de 0,066 mm.

6. Tige de cathéter selon la revendication 1, dans laquelle la couche de cathéter interne et la couche de gaine externe ont la même longueur.

7. Tige de cathéter selon la revendication 1, dans laquelle la première couche intermédiaire en spire et la seconde couche intermédiaire tressée sont encapsulées à l'intérieur à la fois de la couche de cathéter interne et de la couche de gaine externe.

8. Tige de cathéter selon la revendication 1, dans laquelle la première couche intermédiaire en spire est constituée d'au moins deux fils enroulés dans une même direction ou dans une direction opposée.

9. Tige de cathéter selon la revendication 1, dans laquelle la seconde couche intermédiaire tressée est disposée entre la première couche intermédiaire en spire et la couche de gaine externe.

10. Tige de cathéter selon la revendication 9, dans laquelle la seconde couche intermédiaire tressée est disposée au moins sur une partie proximale et une partie médiane de la première couche intermédiaire en spire, éventuellement dans laquelle la seconde couche intermédiaire tressée est au moins 20 mm plus courte que la partie médiane de la première couche intermédiaire en spire.

11. Tige de cathéter selon la revendication 1, dans laquelle la tige de cathéter présente un rapport diamètre externe/diamètre luminal interne d'au moins 1,2:1.

12. Tige de cathéter selon la revendication 1, dans laquelle la première couche intermédiaire en spire présente un pas plus faible dans une partie proximale de la tige de cathéter, et un pas plus élevé dans une partie distale du cathéter, et une variation progressive entre les deux.

13. Tige de cathéter selon la revendication 1, dans laquelle la première couche intermédiaire en spire est disposée sur la couche de cathéter interne ultra-mince, la seconde couche intermédiaire tressée est disposée sur la première couche intermédiaire en spire, et la couche de gaine externe est disposée sur la seconde couche intermédiaire tressée, et dans laquelle la couche de gaine externe est fondue et liée à la couche interne ultra-mince à travers la première couche intermédiaire en spire et la seconde couche intermédiaire tressée.
